(19) Europäisches Patentamt
European Patent Office
Office européen des brevets

(11) **EP 3 143 592 B1**

(12) **EUROPÄISCHE PATENTSCHRIFT**

(45) Veröffentlichungstag und Bekanntmachung des Hinweises auf die Patenterteilung:
**05.12.2018 Patentblatt 2018/49**

(21) Anmeldenummer: **15725254.5**

(22) Anmeldetag: **12.05.2015**

(51) Int Cl.:
***G06T 11/00*** *(2006.01)*

(86) Internationale Anmeldenummer:
**PCT/EP2015/060493**

(87) Internationale Veröffentlichungsnummer:
**WO 2015/173251 (19.11.2015 Gazette 2015/46)**

(54) **VERFAHREN UND VORRICHTUNG ZUR REDUKTION VON ARTEFAKTEN IN COMPUTERTOMOGRAPHISCHEN BILDERN**

METHOD AND APPARATUS FOR REDUCING ARTEFACTS IN COMPUTED TOMOGRAPHY IMAGES

PROCÉDÉ ET DISPOSITIF DE RÉDUCTION D'ARTÉFACTS DANS DES IMAGES OBTENUES PAR TOMODENSITOMÉTRIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.05.2014 DE 102014007095**

(43) Veröffentlichungstag der Anmeldung:
**22.03.2017 Patentblatt 2017/12**

(73) Patentinhaber: **Universität zu Lübeck**
**23562 Lübeck (DE)**

(72) Erfinder:
• **STILLE, Maik**
**23564 Lübeck (DE)**
• **BUZUG, Thorsten M.**
**23627 Gross Sarau (DE)**

(74) Vertreter: **Wallinger, Michael**
**Wallinger Ricker Schlotter Tostmann Patent- und Rechtsanwälte Partnerschaft mbB Zweibrückenstrasse 5-7 80331 München (DE)**

(56) Entgegenhaltungen:
**US-B1- 8 233 586**

• **WEBSTER STAYMAN J ET AL: "Likelihood-based CT Reconstruction of Objects Containing Known Components", 11TH INTERNATIONAL MEETING ON FULLY THREE-DIMENSIONAL IMAGE RECONSTRUCTION IN RADIOLOGY AND NUCLEAR MEDICINE, JULY 11 - JULY 15, 2011, POTSDAM, GERMANY,, 11. Juli 2011 (2011-07-11), Seiten 254-257, XP002696373,**

**Beschreibung**

Die Erfindung betrifft ein Verfahren sowie eine Vorrichtung zur Reduktion von Artefakten, die insbesondere von Störkörpern und/oder Metallkörpern in computertomographischen (CT) Bildern verursacht werden, nach dem Oberbegriff der unabhängigen Ansprüche.

Die Computertomographie (CT) stellt einen erheblichen Fortschritt gegenüber der konventionellen Radiographie dar. Die CT-Bildgebung basiert auf der unterschiedlichen Abschwächung von Röntgenstrahlung durch ein Objekt, wobei aus einer Vielzahl von in verschiedenen Richtungen aufgenommenen Röntgenaufnahmen des Objekts mittels computerbasierter Auswertung Schnittbilder erzeugt werden. Die Bilderzeugung ist schnell, und die Auflösung sowie die Orts- und Lagebestimmung von Körpern ist besser als in der konventionellen Radiographie, bei welcher lediglich ein Projektionsbild des jeweils untersuchten Objekts in einer Ebene erhalten wird. So ist es mittels Computertomographie insbesondere möglich, eine dreidimensionale Darstellung von vaskulären und skelettartigen Strukturen mit hoher, isotroper Ortsauflösung zu gewinnen. Die Computertomographie ist daher eines der bedeutendsten bildgebenden Verfahren im klinischen Alltag.

Ein Problem für das CT-Verfahren stellen Metallkörper im zu untersuchenden Objekt dar, die das bildgebende Verfahren stark stören und zu Artefakten im CT-Bild führen.

So weisen z.B. Zahnfüllungen oder Implantate einen sehr hohen Abschwächungskoeffizienten für Röntgenstrahlung auf. Ab einer gewissen Metalldicke oder im Falle von Kanten und komplexen Geometrien eines Störkörpers kann es zu einer so starken Absorption der Röntgenstrahlung kommen, durch die das Nutzsignal so stark abgeschwächt wird, dass dieses unter der sogenannten Rauschschwelle liegt und damit für eine Differenzierung des Objekts nicht mehr ausreicht.

Eine weitere Störung stellen sogenannte Aufhärtungsartefakte dar. Da die Strahlenabsorption von Objekten von der Energie abhängig ist und niederenergetische Strahlen stärker absorbiert werden als höherenergetische Strahlen, führt dies zu einer Erhöhung der mittleren Energie des Gesamtspektrums der Röntgenstrahlung. Dieser Vorgang tritt bei allen Gewebearten auf, ist jedoch bei Metallen um ein Vielfaches höher und führt zu entsprechenden Fehlern in den CT-Bildern.

In den Projektionen einer computertomographischen Aufnahme zeigen sich abhängig vom Material und der geometrischen Struktur von Metallobjekten im zu untersuchenden Objekt resultierende Metallartefakte als Kombination der genannten physikalischen Effekte als Störeinflüsse. Die entstehenden Artefakte beeinträchtigen den Informationsgehalt des rekonstruierten CT-Bildes bis hin zu dem Punkt, dass die relevante Information zum Teil oder sogar völlig verloren geht und damit eine nachfolgende diagnostische Interpretation der Daten unmöglich wird.

US Patent Nr. 8,233,586 offenbart ein iteratives Verfahren zur Reduzierung von Metallartefakten.

Es ist Aufgabe der Erfindung, ein Verfahren sowie eine entsprechende Vorrichtung anzugeben, durch welches bzw. welche Artefakte in CT-Bildern einfach und zuverlässig reduziert werden.

Diese Aufgabe wird durch das Verfahren und die Vorrichtung gemäß den unabhängigen Ansprüchen gelöst.

Die Erfindung basiert auf dem Ansatz, dass Informationen über Form und/oder Zusammensetzung eines im zu untersuchenden Objekt befindlichen Störkörpers genutzt werden können, um Artefakte im rekonstruierten Bild zu unterdrücken oder zumindest zu reduzieren und so den Detailgehalt des CT-Bildes zu verbessern. Dazu werden in einem Iterationsprozess bzw. iterativen Regelkreis CT-Bilder des zu untersuchenden Objekts, das sowohl bekannte als auch unbekannte Störkörper aus metallischen oder teilweise metallischen Materialien enthalten kann, unter Berücksichtigung von gespeicherten Informationen über den bzw. die Störkörper rekonstruiert, indem insbesondere die durch den bzw. die Störkörper beeinflussten Daten gezielt eliminiert oder zumindest reduziert werden. Das erfindungsgemäße Verfahren bzw. die erfindungsgemäße Vorrichtung beinhaltet also einen iterativen Rekonstruktionsansatz, in den Vorwissen in Form von Nebenbedingungen bei der Reduzierung von Metallartefakten mit einfließt. Vorzugsweise sind Art und Herstellungsmaterial von Implantaten bekannt, datentechnisch erfasst bzw. hinreichend dokumentiert und in einer Datenbank gespeichert. Implantate, welche sich bereits im Körper eines Patienten befinden, sind vorzugsweise in einer Patientenakte datentechnisch erfasst, physikalisch parametrisierbar, abgleichbar und jederzeit abrufbar. Diese Informationen werden vorzugsweise in Form von physikalisch eindeutigen Größen, wie z.B. Abschwächungskoeffizienten, parametrisiert, welche insbesondere die chemische Zusammensetzung und die geometrische Form des Implantates informativ abbilden und im erfindungsgemäßen Rekonstruktionsverfahren berücksichtigt werden, um so den Einfluss der Metallartefakte zu reduzieren bzw. völlig aufzuheben.

Insgesamt werden durch die Erfindung Artefakte in CT-Bildern auf einfache und zuverlässige Weise reduziert.

Im Zusammenhang mit der Erfindung werden unter dem Begriff "Projektionen" mehrere, insbesondere eine Vielzahl von, Absorptionsprofilen des Objekts aus verschiedenen Richtungen verstanden. Dementsprechend sind unter dem Begriff "Projektionsdaten" die das Absorptionsverhalten des Objekts in verschiedenen Richtungen charakterisierenden Daten von Projektionen zu verstehen.

Ferner wird im Zusammenhang mit der Erfindung unter den Begriffen "Bild" und "CT-Bild" eine aus mehreren, insbesondere einer Vielzahl von, Projektionen rekonstruierte Schnitt- oder Volumendarstellung des Objekts und/oder ein entsprechender zwei- bzw. dreidimensionaler Bilddatensatz verstanden, in dem die Information Pixeln bzw. Voxeln zuordnet ist.

Bei bevorzugten Ausgestaltungen der Erfindung werden eine oder mehrere der folgenden Methoden eingesetzt: Filterung von Zwischenergebnissen zur Artefaktreduzierung; dynamisches Gewichten von Nebenbedingungen in einem iterativen Regelkreis; Korrigieren von Inkonsistenzen im Radonraum; Einbringen von bekannten Geometrien von metallischen Störkörpern in das Rekonstruktionsverfahren; Einbringen von bekannten Abschwächungskoeffizienten von metallischen Störkörpern in das Rekonstruktionsverfahren.

Eine oder mehrere der vorstehend genannten Methoden erlauben es, ein Höchstmaß an Informationsdichte aus CT-Messungen und bekannten Patientendaten zu generieren und dem Anwender, insbesondere einem Mediziner, visualisiert zur Verfügung zu stellen. Ferner wird erreicht, technisches Vorwissen über Implantate und deren technisch bekannte parametrisierte Eigenschaften in die Metallartefaktreduzierung (MAR) mit einzubringen. Auch ist es möglich, eine Differenzierung und Analyse der Entstehung von Neuartefakten durch Interpolationsfehler bei bekannten MAR-Ansätzen zu nutzen und in das erfinderische Verfahren mit zu integrieren. Nicht zuletzt ist es möglich, eine Minimierung von Restartefakten während der Rekonstruktion durchzuführen.

In dem weiteren Modul werden Verfahrensschritte zur Verarbeitung der im Rahmen der Messung erhobenen Daten verifiziert und datentechnisch verarbeitet. Dabei ist das eigenständige Modul des erfindungsgemäßen Iterationsprozesses bzw. iterativen Regelkreises in das weitere Modul integrierbar ausgeführt, d.h. alle Schnittstellen und Parameterübergaben sind eindeutig definierbar und damit nicht proprietär, d.h. datentechnisch und messgrößentechnisch gehorchen die Schnittstellen bzw. die zu übergebenen Parameter und Daten international gültigen Standards und Normen und sind damit einfach auch in existierenden Verfahren einsetzbar, die standardisiert angeboten werden.

In dem übergeordneten Modul wird eine Eingabe mit Datenabgleich zu datenbankgestützten parametrisierten Daten und der Abgleich und die Archivierung des Verfahrensergebnisses vorgenommen. Dabei ist auch das übergeordnete Modul in das weitere Modul und eigenständige Modul des erfindungsgemäßen Iterationsprozesses bzw. iterative Regelkreises integrierbar. Für die Schnittstellen und Parameterübergaben gilt das zuvor im Zusammenhang mit dem weiteren Modul Gesagte analog.

Der mehrschichtige Aufbau des Verfahrens bzw. der Vorrichtung aus unterschiedlichen Modulen in Form eines parametrisierten iterativ arbeitenden Regelkreises, eines weiteren Moduls zur Verarbeitung der im Rahmen der Messung erhobenen Daten und eines übergeordneten Moduls, das im Wesentlichen die Dateneingabe und/oder-ausgabe mit einer Datenbank koppelt, erlaubt die datentechnische Adaption weiterer Verfahren in den jeweiligen Modulen, ohne dass die Struktur des Verfahrens bzw. der Vorrichtung einen Bruch in der systematischen Vorgehensweise erfährt.

Weitere bevorzugte Aspekte des Verfahrens bzw. der Vorrichtung zur Reduktion von Artefakten werden im Folgenden anhand eines Beispiels veranschaulicht.

Nach der Identifikation eines Patienten mit dem Namen "Vorname Name" und dem Geburtsdatum "1. April 1979", sogenannten Personendaten, die als Metadaten vorliegen, wird vorzugsweise anhand einer elektronischen Patientenakte zunächst festgestellt, dass bei diesem Patienten ein Metallnagel im Unterschenkel implantiert wurde. Der Metallnagel hat die Modellnummer "N123456", anhand welcher in der Patientenakte oder durch Verlinkung mit einer anderen Datenbank Informationen über die Form, z.B. Länge und Durchmesser, die Zusammensetzung, z.B. Titan, sowie die Abschwächungskoeffizienten des implantierten Nagels abgerufen werden.

Sodann erfolgt ein CT-Scan des Patienten, bei welchem vom Unterschenkel des Patienten eine Vielzahl von ersten Projektionen aufgenommen wird, welche das Absorptionsverhalten der durchdrungenen Materie des Unterschenkels entlang der unterschiedlichen Projektionsrichtungen wiedergeben.

Vor, während oder nach dem CT-Scan werden aus den Daten, die den Metallnagel charakterisieren, Störkörperdaten in Form eines Modells des Metallnagels generiert.

Nach dem CT-Scan erfolgt vorzugsweise eine Verifikation, bei der die Projektionen des CT-Scans ermittelt werden, in welchen der Metallnagel enthalten ist. Projektionen, in denen der Metallnagel detektiert wird, werden in einem ersten Korrekturschritt entfernt und/oder korrigiert, beispielsweise durch Interpolation, Filterung oder Maskierung.

Die bei diesem ersten Korrekturschritt erhaltenen ersten Projektionsdaten werden einem Iterationsprozess bzw. einem iterativen Regelkreis zugeführt, in welchem zunächst aus den ersten Projektionsdaten CT-Bilddaten, insbesondere Schnitt- oder Volumenbilder, des Unterschenkels generiert werden, beispielsweise durch einfache Rückprojektion oder eine sog. gefilterte Rückprojektion (filtered backprojection).

Sodann wird vorzugsweise geprüft, ob die generierten CT-Bilddaten vorgegebene Grenzwerte und/oder Abbruchbedingungen des Iterationsprozesses erfüllen. Dies kann beispielsweise anhand des Gradienten einer Zielfunktion oder einer sog. Log-Likelihood-Funktion für ein Transmissions-CT geschehen.

Bejahendenfalls wird der Iterationsprozess abgebrochen und es erfolgt eine Ausgabe der CT-Bilddaten in Form eines artefaktfreien oder zumindest artefaktreduzierten Bildes des Unterschenkels, das vorzugsweise zur elektronischen Patientenakte des Patienten "Vorname, Name" hinzugefügt bzw. vorhandenen Datensätzen ergänzend zugeordnet werden kann.

Andernfalls wird der Iterationsprozess fortgesetzt, indem eine Reduktion von durch den Metallnagel verursachten Artefakten in den rekonstruierten bzw. generierten CT-Bilddaten durch Filterung der CT-Bilddaten durchgeführt wird. Eine Filterung erfolgt beispielsweise mittels bilateraler Filter, bei welchen die CT-Bilder hinsichtlich Grauwerten und Abständen der einzelnen Pixel bzw. Voxel zueinander gefiltert werden, oder mittels Weichzeichner, wie z.B. Gaußfilter oder Medianfilter. Hierbei werden vom Metallnagel verursachte Artefakte reduziert, wobei gleichzeitig relevante anatomische Strukturen des Unterschenkels erhalten bleiben.

In einem weiteren Schritt werden zweite Projektionsdaten aus den gefilterten CT-Bilddaten generiert. Vorzugsweise wird hierbei eine zur vorstehend beschriebenen Rekonstruktion von CT-Bilddaten aus den ersten Projektionsdaten des Unterschenkels analoge Projektionsmethode verwendet, damit die ersten und zweiten Projektionsdaten zueinander passen.

In einem nachfolgenden Schritt werden die beim CT-Scan des Unterschenkels erhaltenen und nachfolgend einer ersten Korrektur unterzogenen ersten Projektionsdaten mit den zweiten Projektionsdaten kombiniert, indem insbesondere einige erste Projektionsdaten unter Berücksichtigung der Störkörperdaten in Form des Modells des Metallnagels durch zweite Projektionsdaten ersetzt werden. Vorzugsweise werden hierbei erste Projektionen, die mit dem Metallnagel assoziiert werden können, entfernt und durch entsprechende zweite Projektionen ersetzt.

Die hierbei erhaltenen kombinierten Projektionsdaten werden dann als erste Projektionsdaten wieder dem ersten Schritt des Iterationsprozesses zugeführt, bei welchem aus den ersten Projektionsdaten CT-Bilddaten, insbesondere Schnitt- oder Volumenbilder, des Unterschenkels generiert werden.

Der Iterationsprozess wird vorzugsweise so lange durchlaufen, bis die jeweils aktuell generierten CT-Bilddaten - wie bereits vorstehend beschrieben - die vorgegebenen Grenzwerte bzw. Abbruchbedingungen des Iterationsprozesses erfüllen und ein von Artefakten freies oder zumindest reduziertes CT-Bild des Unterschenkels ausgegeben wird.

Weitere Vorteile, Merkmale und Anwendungsmöglichkeiten der vorliegenden Erfindung ergeben sich aus der nachfolgenden Beschreibung in Zusammenhang mit den Figuren. Es zeigen:

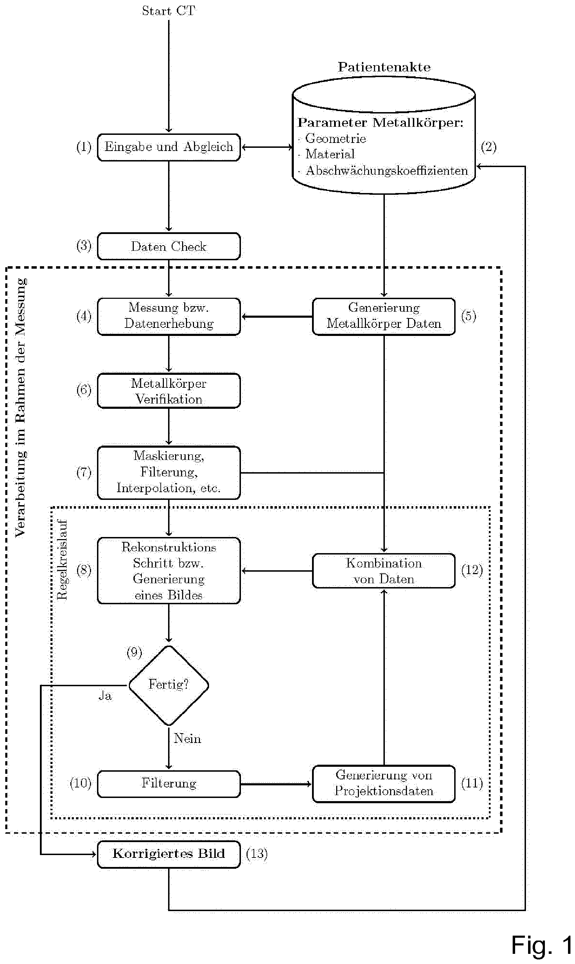

Fig. 1    ein Flussdiagramm für ein Verfahren zur Reduktion von Artefakten, die insbesondere von Störkörpern und/oder Metallkörpern in computertomographischen (CT) Bildern verursacht werden, durch einen geregelten Iterationsprozess, der in einen Prozess zur Verarbeitung von Messdaten und in einen übergeordneten Prozess zum Datenabgleich integriert ist;

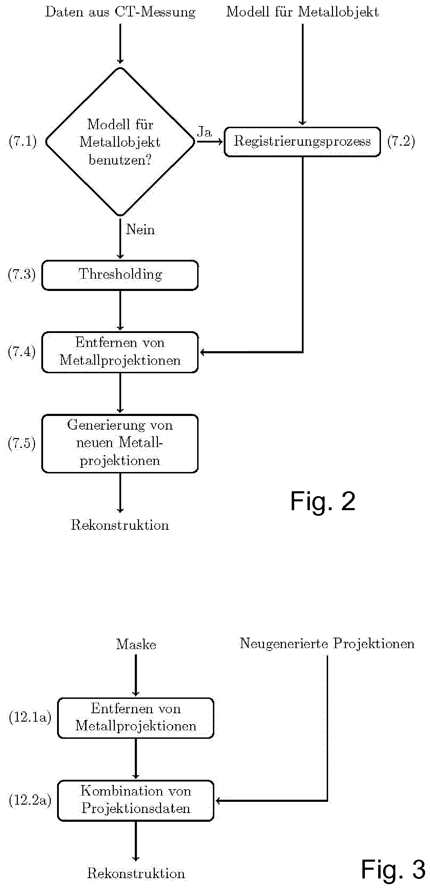

Fig. 2    eine Feinstruktur von Arbeitsschritten aus dem Flussdiagramm der Fig. 1 für ein Verfahren zur Reduktion von Artefakten, die insbesondere von Störkörpern und/oder Metallkörpern in computertomographischen (CT) Bildern verursacht werden, die einen möglichen Prozess zur Verarbeitung von Messdaten zeigen;

Fig. 3    eine Feinstruktur von Arbeitsschritten aus dem Flussdiagramm der Fig. 1 für ein Verfahren zur Reduktion von Artefakten, die insbesondere von Störkörpern und/oder Metallkörpern in computertomographischen (CT) Bildern verursacht werden, die einen möglichen ersten Prozess der Kombination von Daten zeigen; und

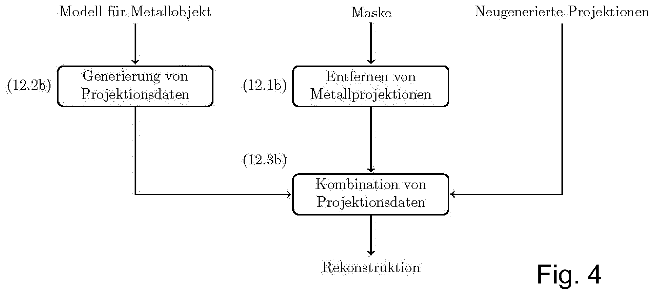

Fig. 4    eine Feinstruktur von Arbeitsschritten aus dem Flussdiagramm der Fig. 1 für ein Verfahren zur Reduktion von Artefakten, die insbesondere von Störkörpern und/oder Metallkörpern in computertomographischen (CT) Bildern verursacht werden, die einen möglichen zweiten Prozess der Kombination von Daten zeigen.

Figur 1 zeigt ein Flussdiagramm für ein Verfahren zur Reduktion von Artefakten, die insbesondere von Störkörpern und/oder Metallkörpern in computertomographischen (CT) Bildern verursacht werden, durch einen geregelten Iterationsprozess (gepunkteter Kasten), der in einen weiteren Prozess zur Verarbeitung von Messdaten (gestrichelter Kasten) und in einen übergeordneten Prozess zum Datenabgleich integriert oder integrierbar ist. Das Verfahren bzw. die entsprechende Vorrichtung ist hierbei in drei Module aufgegliedert. Im ersten Modul sind vier Elemente (1), (2), (3) und (13) vorgesehen, die die Dateneingabe, Datenverwaltung und Datenausgabe umfassen. Das zweite Modul mit den Elementen (4) bis (7) umfasst die Verarbeitung im Rahmen der CT- Messung und das dritte Regelkreis-Modul (Elemente (8) bis (12)), in welchem auf iterativem Wege eine automatisierte Optimierung Bildbearbeitung erfolgt.

Im ersten Element (1) des Flussdiagramms erfolgt die Eingabe und der Abgleich von relevanten Daten. In der Eingabe werden Personendaten, wie zum Beispiel Name, Geburtsdatum, die zur Identifikation dienen, aufgenommen. Es erfolgt ein Abgleich bzw. Zurückgreifen auf Daten aus einer elektronischen Patientenakte oder das Anlegen einer elektronischen Patientenakte (2).

Die elektronische Patientenakte (2) verfügt beispielsweise mindestens über Informationen zum Alter, Krankheitsstatus, den verordneten Medikamenten, der Aufenthaltsdauer eines Patienten und vorzugsweise über Informationen zu vorhandenen Implantaten, insbesondere Metallimplantaten. Sollte ein Metallimplantat vorhanden sein, so kann der Akte beispielsweise die Modellnummer des Implantates entnommen werden. Mit Hilfe dieser Nummer ist es zum Beispiel möglich, Informationen bezüglich Form und/oder Zusammensetzung des Implantates zu erhalten und vorzuhalten und im dritten Element Daten Check (3) zu ergänzen bzw. zu korrigieren.

Das zweite Modul umfasst die Verarbeitung der Daten im Rahmen der Messung. Dazu beinhaltet das vierte Element die Messung bzw. Datenerhebung (4) mit der Durchführung des eigentlichen CT-Scans. Dies umfasst in der Regel eine einmalige Bestrahlung des Patienten mit Erhebung von Rohdaten, insbesondere eine Vielzahl von Projektionen, die das Absorptionsverhalten des Objekts in verschiedenen Richtungen widerspiegeln. Parallel erfolgt eine Generierung der Metallkörper-Daten (5) unter Berücksichtung bzw. auf der Basis von Informationen aus der Patientenakte. Dabei wird vorzugsweise ein Modell für vorhandene Metallimplantate, welche sich im Inneren des Patienten befinden, erstellt. Dieses Modell wird im Weiteren dazu verwendet, auftretende Artefakte zu reduzieren. Insbesondere enthält dieses Modell Informationen über die Geometrie eines Implantates, über dessen chemische Zusammensetzung und über die dadurch resultierenden Abschwächungskoeffizienten.

Im sechsten Element erfolgt eine Metallkörper-Verifikation (6). Anhand der von Element (4) erhaltenen Daten kann dabei festgestellt werden, ob ein Metallkörper im Patienten vorhanden ist oder nicht. Insbesondere kann dies für jede Schicht und/oder Projektion des erfolgten CT-Scans durchgeführt werden. Bei Schichten bzw. Projektionen, die keine Metallobjekte enthalten, bedarf es keiner Metallartefaktkorrektur oder -reduktion. Sollte ein Metallobjekt vorhanden sein, wird mit der vorgeschlagenen Methodik weiter verfahren.

Anschließend erfolgt im siebten Element eine Metallartefaktkorrektur-oder reduktion (MAR) durch Maskierung, Filterung, Interpolation etc. (7), wodurch ein Entfernen bzw. eine Bearbeitung von Projektionen, die durch Metall verlaufen, erreicht wird. Dabei können verschiedene Methoden eingesetzt werden, wie zum Beispiel: lineare Interpolation, quadratische oder kubische Interpolation, B-spline Interpolation oder andere Interpolations-Verfahren, Korrekturverfahren, welche auf Inpainting beruhen, oder Normalized Metal Artifact Reduction.

Das dritte Modul ist im zweiten Modul eingebettet und als eigenständiger, insbesondere iterativer, Regelkreis ausgebildet. In diesem Regelkreis erfolgt im achten Element der Rekonstruktionsschritt (8). Beim erstmaligen Erreichen dieses Elementes wird ein erstes Bild aus den erhobenen Daten generiert. Dies ist in der Regel noch weit entfernt von dem korrigierten Ergebnis in Element (13).

Zur Generierung des Bildes auf Basis der Rohdaten werden folgende Methoden bevorzugt.

Bei einem iterativen Schema ist eine einfache Rückprojektion einsetzbar. Dies beinhaltet z.B. eine Line-driven oder auch Raycasting Projektionsmethode.

Eine analytische Methode, wie zum Beispiel die Filtered Backprojektion, generiert bereits nach einer ersten Iteration ein vollständig rekonstruiertes Bild. Dies kann jedoch noch Artefakte enthalten, welche erst durch mehrfaches Durchlaufen des Regelkreises beseitigt werden.

Eine Terminierungsabfrage (9) im neunten Element stellt fest, ob die Kriterien für die Rekonstruktion anhand vorgegebener Werte erfüllt sind und die Rekonstruktion abgeschlossen werden kann. Dies kann zum Beispiel an Hand des Gradienten einer Zielfunktion geschehen. Im Falle einer statistischen Rekonstruktion kann dies die Log-Likelihood Funktion für ein Transmissions-CT sein. Des Weiteren kann eine Abhängigkeit in Bezug auf das bereits bekannte Vorwissen integriert und die vorgegebenen Werte aus der Eingabe (1) berücksichtigt werden. Hierbei kann z.B. auch überprüft werden, wie exakt die bereits bekannten Abschwächungskoeffizienten im rekonstruierten Bild angenommen wurden. Sind die vorher definierten Terminierungskriterien erfüllt, so ist das Verfahren erfolgreich abgeschlossen und ein fertiges, von Artefakt bereinigtes Bild wird ausgegeben, wie durch das dreizehnte Element (13) veranschaulicht.

Sollten die Kriterien dagegen nicht erfüllt sein, so wird im zehnten Element des Regelkreises eine Filterung (10) durchgeführt. Dabei wird das aktuelle rekonstruierte Bild aus Element (8) mit Bildfiltern, z.B. bilateralen Filtern, verändert. Hier bei wird das Bild hinsichtlich der Grauwerte und der Abstände der einzelnen Pixel bzw. Voxel zueinander gefiltert. Andere Möglichkeiten der Filterung sind durch Weichzeichner, wie zum Beispiel Gauß-Filter oder Median-Filter, gegeben. Dabei ist der Regelkreis derart parametrisiert, dass Artefakte reduziert werden, jedoch anatomische Strukturen des Patienten erhalten bleiben.

Im elften Element wird eine Generierung von Projektionsdaten (11) vorgenommen. Damit die Projektionsdaten zueinander passen, wird für die Generierung von neuen Projektionsdaten dieselbe Projektionsmethode benutzt, wie im achten Element (8). Durch die Generierung von Projektionsdaten wird das Verhalten des CT-Gerätes, welches im vierten Element (4) die Messung durchführt, simuliert. Dementsprechend wird hierbei zum Erzeugen der Projektionsdaten der Patient nicht nochmals mit Röntgenstrahlen belastet, sondern das aktuell rekonstruierte Bild wird herangezogen und der Verlauf der Strahlen wird simuliert. Die generierten Projektionsdaten werden im zwölften Element (12) weiter verarbeitet, indem eine Kombination von Daten (12) erfolgt. Dabei werden Projektionsdaten, welche durch die Methodik in Element (7) erzeugt wurden, durch die Projektionsdaten welche in Element (11) generiert wurden, ersetzt. Hierzu kann die Maske, welche im Element (7) verwendet wurde, ebenfalls benutzt werden. Projektionen, welche mit dem Metallobjekt assoziiert werden, werden zunächst entfernt und durch die in Element (11) generierten Projektionen ersetzt. Eine Kombination der aus Element (7) und (11) resultierenden Projektionen ist hierbei möglich.

Im dreizehnten Element wird der Regelkreis verlassen und ein korrigiertes Bild (13) als Ergebnis des Verfahrens erzeugt bzw. ausgegeben. Das Verfahren erzeugt als Rekonstruktion ein artefaktfreies oder zumindest artefaktreduziertes Bild, welches anschließend der elektronischen Patientenakte (2) hinzugefügt wird, um die Informationsdichte zu verbessern.

Figur 2 zeigt ein beispielhaftes Verfahren zur Ausgestaltung des siebten Elements (7) aus Figur 1, insbesondere für die gezielte Maskierung, Filterung und Interpolation.

In Element (7.1) wird durch eine Datenbankabfrage entschieden, ob ein parametrisiertes Modell für das Metallobjekt aus der CT-Messung vorhanden ist und ob dieses benutzt werden soll. Die Kriterien für diese Entscheidung sind in der Datenbank abgelegt. Ist ein Modell des Metallobjektes für die Maskierung bzw. Segmentierung in der Datenbank abgelegt und damit parametrisiert abrufbar, so wird dies durch einen Registrierungsschritt (7.2) verifiziert, das Registrierungsproblem "Finde die korrekte Position des Metallobjektes im Bild" gelöst und eine exakte Segmentierung des Metallobjekts vorgenommen. Diese Segmentierung kann im Regelkreis bei jedem weiteren Durchlauf erfolgen und damit durch Iteration verfeinert werden.

Es gibt für das weitere Abarbeiten der Schritte im Verfahren eine Fallunterscheidung.

Soll das erkannte Modell für das Metallobjekt nicht benutzt werden, zum Beispiel um die zusätzliche Laufzeit durch den Registrierungsprozess zu vermeiden, so muss die Klassifikation von Metall im Bild durch ein anderes Verfahren im Element Thresholding (7.3) gelöst werden. Ein Beispiel für eine derartige Vorgehensweise durch dieses Element ist eine sogenannte Threshold-Segmentierung. Dabei wird ein vorbestimmter Schwellenwert für die Parametrisierung von Metallen festgelegt, der das Bild in zwei Bereiche mit den Kriterien "Metall" und "NichtMetall" einteilt.

Im Element (7.4) "Entfernen von Metallprojektionen", d.h. von Projektionen bzw. Röntgenstrahlen, welche durch Metall verlaufen, wird damit eine Segmentierung des Metallobjektes vorgenommen. Alle Projektionsdaten, welche mit dem bestimmten Metallobjekt datentechnisch assoziiert sind, werden aus den im Original gemessenen Daten entfernt. Bei unspezifizierten Daten, bei denen z.B. nicht unterschieden wurde, ob eine Segmentierung im Rohdatenraum oder im Bildbereich vorgenommen wurde, wird hier eine weitere parametrisierte Fallunterscheidung durchgeführt. Sollte die Einteilung im Bildbereich vorgenommen worden sein, so muss eine Projektion der resultierenden Maske für das Metallobjekt in den Rohdatenraum vollzogen werden.

Im Falle des Vorhandenseins einer Einteilung von Projektionen in Metallprojektionen und Nicht-Metallprojektionen, können die Projektionen, welche mit Metall assoziiert werden, aus dem Rohdatenraum bzw. aus den gemessen Daten entfernt werden.

Durch das Entfernen der Metallprojektionen entsteht eine Lücke im Rohdatenraum bzw. in den gemessen Daten. Dies bedeutet, dass Informationen, welche für die Rekonstruktion eines Bildes benötigt werden, fehlen. Eine Möglichkeit ist es, die Rekonstruktion unter Ausschluss der Metallprojektionen durchzuführen. Dies erzeugt Artefakte im Bild, welche bereinigt werden. Eine andere Möglichkeit besteht darin, eine initiale Metallartefaktreduzierung durchzuführen. Hierbei wird die entstandene Lücke im Element (7.5) durch neu generierte Metallprojektionen ersetzt. Diese könnten zum Beispiel im einfachsten Falle aus einer linearen Interpolation resultieren. Zu bemerken ist, dass dieser Schritt sehr variabel ist und eine Vielzahl an Methoden benutzt werden können.

Figur 3 und 4 zeigen mögliche Verfahren zur Regelung des zwölften Elements (12) aus Figur 1 zur Kombination von Daten.

In Figur 3 ist eine erste Variante für einen Regelschritt in Element (12) des Verfahrens aus Figur 1 erläutert. Das Element "Entfernen von Metallprojektionen" (12.1a) entfernt unter Verwendung der Maske, welche in Element (7) entstanden ist bzw. verwendet wurde, die Metallprojektionen aus den gemessen Daten. Sollten die Kriterien für die Projektionsdaten in Element (12.2a) erfüllt sein, werden die Daten im Element (12.2a) zwischengespeichert.

Die aus dem Element "Generierung von Projektionsdaten" (11) generierten Projektionsdaten, die durch das Metallobjekt verlaufen, werden im Element "Kombination von Projektionsdaten" (12.2a) mit den gemessenen Projektionsdaten kombiniert. Das Ergebnis besteht damit aus den in Element (4) gemessen Projektionen, welche mit der Anatomie des Patienten assoziiert werden, und den im Element (11) künstlich generierten Projektionen.

Die neuen Metallprojektionen bestehen zum Beispiel aus einer gewichteten Kombination von original in Element (4) gemessenen, in Element (7) generierten und in Element (11) erhaltenen Projektionen. Auch eine ausschließliche Nutzung der Projektionen, welche aus Element (11) resultieren, ist damit möglich.

In Figur 4 ist eine Variante für einen Regelschritt in Element (12) des Verfahrens aus Figur 1 erläutert. Zunächst werden unter Verwendung der Maske aus Element (7) die Metallprojektionen aus den gemessen Daten in Element (12.1b) entfernt. Sollten die Kriterien für die Projektionsdaten in Element (12.2b) erfüllt sein, werden die Daten im Element (12.2b) zwischengespeichert. Unter Verwendung des in Element (5) generierten Modells für das Metallimplantat werden in Element (12.2b) neue Projektionsdaten generiert. Das Modell enthält Informationen über die Geometrie und Zusammensetzung des Implantats. Hieraus lassen sich korrekte Abschwächungskoeffizienten ableiten, welche wiederum mit Hilfe einer Vorwärtsprojektion zu korrekten Projektionswerten führen.

Zusammen mit den Projektionen, welche aus Element (11) generiert wurden, und den in Element (12.2b) erzeugten Projektionen wird in Element (12.3b) eine Kombination von Projektionswerten erstellt. Diese Menge an neuen Projektionen kann die Lücke im Rohdatenraum schließen. So kann ein kompletter Datensatz für die Rekonstruktion herangezogen werden. Bei der Erstellung der Projektionen in Element (11) werden die Informationen über das Metallobjekt jeweils abgeglichen, damit keine Fehlinformationen oder Doppel zu künstlichen Artefakten führen.

Als ein Beispiel für ein Rekonstruktionsverfahren wird im Folgenden ein Maximum-Likelihood-Algorithmus für eine iterative Rekonstruktion von CT-Bildern näher beschrieben.

Der Algorithmus basiert auf der Annahme, dass Strahlenquanten, welche von individuellen Detektorelementen gemessen werden, einer Poisson-Statistik unterliegen. Dabei wird die negierte Log-Likelihood-Funktion wie folgt definiert:

$$l(f) = \sum_{i=1}^{M} \left( -n_i \ln(n_0) + n_i \sum_{j=1}^{N} a_{ij} f_j + \ln(n_i!) + n_0 \exp\left(- \sum_{j=1}^{N} a_{ij} f_j\right)\right), \qquad (1)$$

wobei $n_0$ der Anzahl an Strahlenquanten entspricht, welche an der Röntgenröhre erzeugt werden, und $n_i$ den gemessenen Strahlenquanten am Detektor i entspricht. $f \in R^N$ ist ein Vektor, der die erwarteten Abschwächungskoeffizienten enthält und dem zu rekonstruierenden Bildvektor gleichzusetzen ist. $N$ entspricht der Anzahl an Pixeln im Bild und $M$ der Anzahl an Detektorelementen.

Durch eine Minimierung der Funktion (1) ist es möglich, ein Bild $f$ vom tomographierten Objekt aus den Intensitätsdaten $n$ zu rekonstruieren. Durch weitere Betrachtung der Gleichung lässt sich feststellen, dass die gemessenen Intensitäten $n_i$ mit $i = 1, ..., M$ und die Anzahl $n_0$ an Strahlenquanten, welche an der Röntgenröhre erzeugt werden, für ein einzelnes Rekonstruktionsproblem konstant sind und im Laufe der Optimierung sich nicht verändern. Folglich muss man konstante Terme für die weitere Betrachtung des Problems nicht weiter berücksichtigen.

Die Normalisierte Gleichung mit der Anzahl an Projektionen M kann vereinfacht dargestellt werden, da die durch metallische Körper verursachten Projektionen, die für das Entstehen von Artefakten verantwortlich sind, bei der Rekonstruktion nicht berücksichtigt werden sollen.

Weiter wird davon ausgegangen, dass die Abschwächungskoeffizienten und die Geometrie des Metallobjektes (in der Medizin beispielsweise Implantate oder Herzschrittmacher), das sich im zu rekonstruierendem Bild befindet, bekannt sind. Dieses Vorwissen lässt sich in die Minimierung einer Funktion in Form einer Nebenbedingung einbringen. Somit ergibt sich ein Optimierungsproblem, welches man wie folgt formulieren kann: "Minimiere die Log-Follikel-Gleichung zur Rekonstruktion von CT-Bildern unter der Nebenbedingung, dass bestimmte Pixel im Bild die bereits bekannten Abschwächungskoeffizienten erhalten".

Das Ergebnis dieser Betrachtung lässt sich in einer Diagonalmatrix darstellen, welche die Position und Geometrie bekannter Metallobjekte abbildet. Die weiteren Informationen über die Abschwächungskoeffizienten des bekannten Metallobjektes sind als Vektor abgelegt.

Das erfinderische Rekonstruktionsverfahren ist ein iteratives selbstregelndes Verfahren, welches vorzugsweise den Augmented-Lagrangian-Ansatz nutzt. Dabei wird zur Rekonstruktion eines CT-Bildes ein Regelverfahren eingesetzt, welches zwischen einem festgestellten minimalen und maximalen Wert für den Abschwächungskoeffizienten bis zu einer vorbestimmten Toleranzgrenze eine eindeutige Lösung bestimmt.

Ein entschiedener Schritt zur Metallartefaktreduzierung wird nach der Aktualisierung der Gewichtungsfaktoren und Toleranzgrenzen ausgeführt. Unterschreitet die aktuelle Toleranzgrenze einen zuvor festgelegten Wert und liegt die Norm der Nebenbedingung unter einem zuvor festgelegten Wert, so werden ab diesem Zeitpunkt Projektionen, welche durch das bekannte Metallobjekt verlaufen, nicht mehr ignoriert. Stattdessen werden neue Projektionen auf Grundlage der aktuellen Iterierten berechnet und in die akquirierten Daten integriert.

Hierbei wird z.B. mit Hilfe eines bilateralen Filters gearbeitet. Ziel ist es hierbei, Artefakte in der aktuellen Iteration zu unterdrücken und dabei jedoch Kanten und Strukturen im Bild zu erhalten. Bilaterale Filter lassen es zu, das Bild in Abhängigkeit einer Spannweite für den Wertebereich und einer definierten Anzahl miteinbezogener Nachbarschaftspixel weich zu zeichnen, um so gezielt Artefakte zu unterdrücken. An die Filterung anschließend werden dann unter Benutzung einer Vorwärtsprojektion Projektionswerte von diesem Bild berechnet. Nun kann die "Lücke" in den Rohdaten gefüllt werden und ein vollständiger Datensatz zur Rekonstruktion herangezogen werden. Dabei wird die Qualität des Bildes mit jeder Iteration verbessert und es werden in jeder Iteration Projektionsdaten berechnet, welche den original aufgenommen Daten detailgetreuer entsprechen und somit für weniger Artefakte sorgen, weil nur akquirierte Projektionsdaten, welche durch Metall verlaufen und daher unbrauchbar sind, ersetzt werden.

**Bezugszeichenliste**

Elemente

1    Eingabe und Abgleich
2    parametrisierte Datenbank

3    Daten Check
4    Messung bzw. Datenerhebung
5    Generierung Metallkörper Daten
6    Metallkörper Verifikation
7    Maskierung, Filterung, Interpolation
8    Rekonstruktion/Generierung eines Bildes
9    Regelung/Terminierungsabfrage
10    Filterung
11    Generierung von Projektionsdaten
12    Kombination von Daten
13    Korrigiertes Bild

**Patentansprüche**

1.  Verfahren zur Reduktion von Artefakten, die insbesondere von Störkörpern und/oder Metallkörpern verursacht werden, in computertomographischen (CT) Bildern, wobei in zumindest einem Iterationsprozess folgende Schrittfolge ausgeführt wird:

    (a) Rekonstruktion oder Generierung von CT-Bilddaten aus ersten Projektionsdaten,
    (b) Abfrage eines oder mehrerer Grenzwerte und/oder Abbruchbedingungen des Iterationsprozesses und, im Falle des Erreichens der Grenzwerte bzw. Erfüllens der Abbruchbedingungen, Abbruch des Iterationsprozesses und Ausgabe der CT-Bilddaten, andernfalls Fortsetzung des Iterationsprozesses mit Schritt (c),
    (c) Reduktion von Artefakten in den rekonstruierten bzw. generierten CT-Bilddaten durch Filterung der CT-Bilddaten,
    (d) Generierung von zweiten Projektionsdaten aus den gefilterten CT-Bilddaten, und
    (e) Kombination der ersten und zweiten Projektionsdaten, insbesondere Ersetzen von ersten Projektionsdaten durch zweite Projektionsdaten, unter Berücksichtigung von Störkörperdaten und Weitergabe der hierbei erhaltenen Projektionsdaten als erste Projektionsdaten an Schritt (a),

    wobei der zumindest eine Iterationsprozess in einen weiteren Prozess integriert ist, der folgende Schritte aufweist:

    (f) Generierung der Störkörperdaten anhand von den Störkörper charakterisierenden Parametern, die in einer elektronischen Patientenakte gespeichert und abrufbar sind, wobei ein Modell des sich im Inneren eines Patienten befindlichen Störkörpers erstellt wird,
    (g) Erfassung bzw. Übernahme von ersten Projektionsdaten und Parametrisierung der ersten Projektionsdaten,
    (h) Überprüfung, ob ein Störkörper im Objekt vorhanden ist, anhand der ersten Projektionsdaten und
    (i) Bearbeitung, insbesondere Maskierung und/oder Filterung und/oder Interpolation, der ersten Projektionsdaten und Übergabe der bearbeiteten ersten Projektionsdaten an Schritt (a),

    wobei der zumindest eine Iterationsprozess zusammen mit dem weiteren Prozess in einen übergeordneten Prozess integriert ist, der folgende Schritte aufweist:

    (j) Start des Verfahrens,
    (k) Eingabe von patientenbezogenen Daten,
    (l) Abrufen der den Störkörper charakterisierenden Parameter aus der elektronischen Patientenakte anhand der eingegebenen patientenbezogenen Daten und
    (m) Weitergabe der abgerufenen Parameter, insbesondere nach einer Überprüfung und gegebenenfalls Ergänzung und/oder Korrektur der Parameter, an Schritt (f).

2.  Vorrichtung zur Reduktion von Artefakten, die insbesondere von Störkörpern und/oder Metallkörpern verursacht werden, in computertomographischen (CT) Bildern, mit zumindest einen iterativen Regelkreis, welcher zur Ausführung folgender Schrittfolge ausgebildet ist:

    (a) Rekonstruktion oder Generierung von CT-Bilddaten aus ersten Projektionsdaten,
    (b) Abfrage eines oder mehrerer Grenzwerte und/oder Abbruchbedingungen des Iterationsprozesses und, im Falle des Erreichens der Grenzwerte bzw. Erfüllens der Abbruchbedingungen, Abbruch des Iterationsprozesses und Ausgabe der CT-Bilddaten, andernfalls Fortsetzung des Iterationsprozesses mit Schritt (c),

(c) Reduktion von Artefakten in den rekonstruierten bzw. generierten CT-Bilddaten durch Filterung der CT-Bilddaten,

(d) Generierung von zweiten Projektionsdaten aus den gefilterten CT-Bilddaten,

(e) Kombination der ersten und zweiten Projektionsdaten, insbesondere Ersetzen von ersten Projektionsdaten durch zweite Projektionsdaten, unter Berücksichtigung von Störkörperdaten und Weitergabe der hierbei erhaltenen Projektionsdaten als erste Projektionsdaten an Schritt (a),

wobei die Schrittfolge in einen weiteren Prozess integriert ist, der folgende Schritte aufweist:

(f) Generierung der Störkörperdaten anhand von den Störkörper charakterisierenden Parametern, die in einer elektronischen Patientenakte gespeichert und abrufbar sind, wobei ein Modell des sich im Inneren eines Patienten befindlichen Störkörpers erstellt wird.

(g) Erfassung bzw. Übernahme von ersten Projektionsdaten und Parametrisierung der ersten Projektionsdaten,

(h) Überprüfung, ob ein Störkörper im Objekt vorhanden ist, anhand der ersten Projektionsdaten, und

(i) Bearbeitung, insbesondere Maskierung und/oder Filterung und/oder Interpolation, der ersten Projektionsdaten und Übergabe der bearbeiteten ersten Projektionsdaten an Schritt (a),

wobei die Schrittfolge zusammen mit dem weiteren Prozess in einen übergeordneten Prozess integriert ist, der folgende Schritte aufweist:

(j) Start des Verfahrens,

(k) Eingabe von patientenbezogenen Daten,

(l) Abrufen der die Störkörper charakterisierenden Parameter aus der elektronischen Patientenakte anhand der eingegebenen patientenbezogenen Daten, und

(m) Weitergabe der abgerufenen Parameter, insbesondere nach einer Überprüfung und gegebenenfalls Ergänzung und/oder Korrektur der Parameter, an Schritt (f).

## Claims

1. A method for reducing artefacts in computed tomography (CT) images caused in particular by obstructive bodies and/or metal bodies, wherein the following sequence of steps is performed in at least one iteration process:

(a) reconstructing or generating CT image data from first projection data,

(b) querying one or more limiting values and/or terminating conditions of the iteration process and, in the case of the limiting values being reached and/or the terminating conditions being met, aborting the iteration process and outputting the CT image data, otherwise continuing the iteration process with step (c),

(c) reducing artefacts in the reconstructed/generated CT image data by filtering the CT image data,

(d) generating second projection data from the filtered CT image data, and

(e) combining the first and second projection data, in particular replacing first projection data by second projection data in consideration of the obstructive body data and transferring the projection data thereby obtained to step (a) as first projection data,

wherein the at least one iteration process is integrated into a further process having the following steps:

(f) generating the obstructive body data on the basis of parameters characterizing the obstructive body which are stored in and retrievable from an electronic patient record, wherein a model of the obstructive body within a patient is created,

(g) collecting/acquiring first projection data and parameterizing said first projection data,

(h) verifying whether an obstructive body is present in the object based on the first projection data, and

(i) processing, in particular masking and/or filtering and/or interpolating the first projection data and transferring the processed first projection data to step (a),

wherein the at least one iteration process together with the further process is integrated into a superordinate process having the following steps:

(j) starting the method,

(k) inputting patient-related data,

(l) retrieving the parameters characterizing the obstructive body from the electronic patient record on the basis of the entered patient-related data,

(m) transferring the retrieved parameters to step (f), in particular subsequent verification and, if applicable, supplementation and/or correction of the parameters.

**2.** An apparatus for reducing artefacts in computed tomography (CT) images caused in particular by obstructive bodies and/or metal bodies by means of at least one iterative control loop which is configured to perform the following sequence of steps:

(a) reconstructing or generating CT image data from first projection data,

(b) querying one or more limiting values and/or terminating conditions of the iteration process and, in the case of the limiting values being reached and/or the terminating conditions being met, aborting the iteration process and outputting the CT image data, otherwise continuing the iteration process with step (c),

(c) reducing artefacts in the reconstructed/generated CT image data by filtering the CT image data,

(d) generating second projection data from the filtered CT image data,

(e) combining the first and second projection data, in particular replacing first projection data by second projection data in consideration of the obstructive body data and transferring the projection data thereby obtained to step (a) as first projection data,

wherein the sequence of steps is integrated into a further process having the following steps:

(f) generating the obstructive body data on the basis of parameters characterizing the obstructive body which are stored in and retrievable from an electronic patient record, wherein a model of the obstructive body within a patient is created,

(g) collecting/acquiring first projection data and parameterizing said first projection data,

(h) verifying whether an obstructive body is present in the object based on the first projection data, and

(i) processing, in particular masking and/or filtering and/or interpolating the first projection data and transferring the processed first projection data to step (a),

wherein the sequence of steps together with the further process is integrated into a superordinate process having the following steps:

(j) starting the method,

(k) inputting patient-related data,

(l) retrieving the parameters characterizing the obstructive body from the electronic patient record on the basis of the entered patient-related data,

(m) transferring the retrieved parameters to step (f), in particular subsequent verification and, if applicable, supplementation and/or correction of the parameters.

**Revendications**

**1.** Procédé servant à réduire des artéfacts, qui sont provoqués en particulier par des corps parasites et/ou des corps métalliques, dans des images tomodensitométriques, dans lequel une succession d'étapes suivante est exécutée lors d'au moins un processus d'itération :

(a) la reconstitution ou la génération de données d'image tomodensitométriques à partir de premières données de projection,

(b) la consultation d'une ou de plusieurs valeurs limites et/ou de critères d'interruption du processus d'itération et, si les valeurs limites sont atteintes ou les critères d'interruption sont remplis, l'interruption du processus d'itération et la sortie des données d'image tomodensitométriques, sinon la poursuite du processus d'itération avec l'étape (c),

(c) la réduction d'artéfacts dans les données d'image tomodensitométriques reconstituées ou générées par filtrage des données tomodensitométriques,

(d) la génération de deuxièmes données de projection à partir des données d'image tomodensitométriques filtrées, et

(e) la combinaison des premières et deuxièmes données de projection, en particulier le remplacement de premières données de projection par des deuxièmes données de projection en tenant compte de données de corps parasites, et le transfert des données de projection obtenues dans le cas présent en tant que premières données de projection à l'étape (a),

dans lequel l'au moins un processus d'itération est intégré dans un autre processus, qui présente des étapes suivantes :

(f) la génération des données de corps parasites à l'aide de paramètres caractérisant des corps parasites, qui sont mémorisés dans un dossier de patient électronique et peuvent être consultés, dans lequel un modèle du corps parasite se trouvant à l'intérieur d'un patient est créé,

(g) la détection ou la réception de premières données de projection et le paramétrage des premières données de projection,

(h) la vérification pour savoir si un corps parasite est présent dans l'objet à l'aide des premières données de projection, et

(i) le traitement, en particulier le masquage et/ou le filtrage et/ou l'interpolation, des premières données de projection et la transmission des premières données de projection traitées à l'étape (a),

dans lequel l'au moins un processus d'itération conjointement avec l'autre processus est intégré dans un processus supérieur, qui présente des étapes suivantes :

(j) le lancement du procédé,

(k) la saisie de données concernant le patient,

(1) la consultation des paramètres caractérisant le corps parasite dans le dossier de patient électronique à l'aide des données saisies se rapportant au patient, et

(m) le transfert des paramètres consultés, en particulier après une vérification et éventuellement un ajout et/ou une correction des paramètres, à l'étape (f).

2. Dispositif servant à réduire des artéfacts, qui sont provoqués en particulier par des corps parasites et/ou des corps métalliques, dans des images tomodensitométriques, avec au moins un circuit de régulation itératif, qui est réalisé pour exécuter la succession d'étapes suivante :

(a) la reconstitution ou la génération de données d'image tomodensitométriques à partir de premières données de projection,

(b) la consultation d'une ou de plusieurs valeurs limites et/ou de critères d'interruption du processus d'itération et, si les valeurs limites sont atteintes ou les critères d'interruption sont remplis, l'interruption du processus d'itération et la sortie des données d'image tomodensitométriques, sinon la poursuite du processus d'itération avec l'étape (c),

(c) la réduction d'artéfacts dans les données d'image tomodensitométriques reconstituées ou générées par filtrage des données d'image tomodensitométriques,

(d) la génération de deuxièmes données de projection à partir des données d'image tomodensitométriques filtrées, et

(e) la combinaison des premières et deuxièmes données de projection, en particulier le remplacement de premières données de projection par des deuxièmes données de projection en tenant compte de données de corps parasites, et le transfert des données de projection obtenues dans le cas présent en tant que premières données de projection à l'étape (a),

dans lequel la succession d'étapes est intégrée dans un autre processus, qui présente des étapes suivantes :

(f) la génération des données de corps parasites à l'aide de paramètres caractérisant des corps parasites, qui sont mémorisés dans un dossier de patient électronique et peuvent être consultés, dans lequel un modèle du corps parasite se trouvant à l'intérieur d'un patient est créé,

(g) la détection ou la réception de premières données de projection et le paramétrage des premières données de projection,

(h) la vérification pour savoir si un corps parasite est présent dans l'objet à l'aide des premières données de projection, et

(i) le traitement, en particulier le masquage et/ou le filtrage et/ou l'interpolation, des premières données de projection, et la transmission des premières données de projection traitées à l'étape (a),

dans lequel la succession d'étapes conjointement avec l'autre processus est intégrée dans un processus supérieur, qui présente des étapes suivantes :

(j) le lancement du procédé,
(k) la saisie de données concernant le patient,
(1) la consultation des paramètres caractérisant les corps parasites dans le dossier de patient électronique à l'aide des données saisies se rapportant au patient, et
(m) le transfert des paramètres consultés, en particulier après une vérification et éventuellement un ajout et/ou une correction des paramètres, à l'étape (f).

Start CT

Patientenakte

(1) Eingabe und Abgleich

Parameter Metallkörper:
· Geometrie
· Material
· Abschwächungskoeffizienten (2)

(3) Daten Check

Verarbeitung im Rahmen der Messung

(4) Messung bzw. Datenerhebung

(5) Generierung Metallkörper Daten

(6) Metallkörper Verifikation

(7) Maskierung, Filterung, Interpolation, etc.

Regelkreislauf

(8) Rekonstruktions Schritt bzw. Generierung eines Bildes

(12) Kombination von Daten

(9) Fertig?

Ja

Nein

(10) Filterung

(11) Generierung von Projektionsdaten

(13) Korrigiertes Bild

Fig. 1

Daten aus CT-Messung          Modell für Metallobjekt

(7.1)    Modell für
         Metallobjekt    Ja    Registrierungsprozess    (7.2)
         benutzen?

         Nein

(7.3)    Thresholding

(7.4)    Entfernen von
         Metallprojektionen

(7.5)    Generierung von
         neuen Metall-
         projektionen

         Rekonstruktion

## Fig. 2

Maske          Neugenerierte Projektionen

(12.1a)    Entfernen von
           Metallprojektionen

(12.2a)    Kombination von
           Projektionsdaten

           Rekonstruktion          **Fig. 3**

Modell für Metallobjekt        Maske        Neugenerierte Projektionen

(12.2b)
```
Generierung von
Projektionsdaten
```

(12.1b)
```
Entfernen von
Metallprojektionen
```

(12.3b)

```
Kombination von
Projektionsdaten
```

Rekonstruktion

# Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8233586 B